# EUROPEAN PATENT APPLICATION

(11) **EP 3 662 963 A1**
(43) Date of publication of application: **10.06.2020**
(21) Application number: 19205520.0
(22) Date of filing: 26.10.2019
(51) Int. Cl.: A61M 27/00, A61M 25/01, A61M 25/00, A61M 29/00

(54) **DRAINAGE CATHETER AND GUIDE WIRE INTRODUCER KIT**

(30) Priority: 30.10.2018 US 201862752424 P; 22.04.2019 US 201962836816 P
(71) Applicant: Demeritt, John S, Saddle River NJ 07458 (US); Toth, Patrick J, New York NY 10075 (US)
(72) Inventor: Demeritt, John S, Saddle River NJ 07458 (US); Toth, Patrick J, New York NY 10075 (US)
(74) Representative: Sessford, Russell

(57) **Abstract**

The present disclosure includes a drainage catheter assembly (10) comprising:

a catheter (12), comprising a flexible, hollow body (20) terminating in a distal end (18) comprising a tapered tip (22) and defining a catheter lumen (21) with a tip outlet (28) in the tapered tip (22);

a hollow stiffening rod (14) slidably received within the catheter lumen (21) and defining a stiffening rod lumen (44) marginally greater than 0.455 millimeters (0.018 inches);

the catheter lumen (21) having a first portion (34) sized to slidably receive the stiffening rod (44) and a second portion (36), at least including the tip outlet (2), and being marginally greater than 0.455 millimeters (0.018 inches); and

at least one fluid port (40) located in the hollow body (20) at the distal end (18).

A guide wire insertion kit including the drainage catheter is also provided.

## Description

### TECHNICAL FIELD

The disclosure generally relates to an apparatus for percutaneous access to the inside of the body. In particular, the invention relates to a drainage catheter assembly, and more particularly, to drainage catheter assembly and kit that includes a catheter that can receive a hollow stiffening rod.

### BACKGROUND

Traditionally, when used for the placement of a drainage catheter, guide wire introducer kits include a 21 gauge needle, a first guide wire with an outside diameter ("OD") of approximately 0.018 inch (0.46 millimeters), a second guide wire with an OD of approximately 0.035 inch (0.90 millimeters) or 0.038 inch (0.97 millimeters), and a coaxial exchange sheath dilator. The exchange sheath dilator includes an outer sheath and an inner dilator mounted to a 21 gauge inner metal cannula. The inner dilator can, for example, have a 4 French OD (0.053 inch, 1.33 millimeters) and an inner diameter ("ID") of approximately 0.028 inches (0.71 millimeters). The outer sheath ID is approximately 4.5 French (0.060 inch, 1.50 millimeters).

The needle can be used to place the first guide wire, then removed, leaving the first guide wire. The exchange sheath dilator can be used to exchange the first guide wire with the second guide wire by sliding the sheath dilator over the first guide wire. The inner dilator and the first guide wire are then removed; leaving the outer sheath. The second guide wire is then inserted through the sheath. Once the second guide wire is placed, the sheath can then be removed so that the second guide wire can be used to place a drainage catheter or an additional sheath dilator, where the drainage catheter or the additional sheath dilator have an ID of approximately 0.035 inch (0.90 millimeters) or .038 inch (0.965 millimeters). Alternatively the second wire can be placed parallel to the first wire prior to removal of the first wire and the outer sheath. The second guide wire is then removed after placement of the additional sheath dilator or drainage catheter. The drainage catheter or the additional sheath dilator tend to be 6 to 10 French or larger and use the larger second guide wire to facilitate placement. The additional sheath dilator that is 6 French to 10 French can provide access to the body for a variety of tools and drainage options.

### SUMMARY

According to one aspect, the disclosure relates to a drainage catheter assembly including a catheter with a flexible, hollow body terminating in a distal end that includes a tapered tip, and the hollow body defining a catheter lumen with a tip outlet in the tapered tip, a hollow stiffening rod slidably received within the catheter lumen and defining a stiffening rod lumen marginally greater than 0.018 inch (0.46 millimeters), the catheter lumen having a first portion sized to slidably receive the stiffening rod and a second portion, at least including the tip outlet, and marginally greater than 0.018 inch (0.46 millimeters), and at least one fluid port located in the hollow body at the distal end.

An aspect provides a drainage catheter assembly comprising: a catheter, comprising a flexible, hollow body terminating in a distal end comprising a tapered tip and the hollow body defining a catheter lumen with a tip outlet in the tapered tip; a hollow stiffening rod slidably received within the catheter lumen and defining a stiffening rod lumen marginally greater than 0.455 millimeters (0.018 inches); the catheter lumen having a first portion sized to slidably receive the stiffening rod and a second portion, at least including the tip outlet, and marginally greater than 0.455 millimeters (0.018 inches); and at least one fluid port located in the hollow body at the distal end.

The at least the distal end may comprise a curled portion.

The curled portion may be straightened when the stiffening rod is received within the catheter lumen.

The at least one fluid port may be located in the curled portion.

The first portion may extend into the tapered tip.

The at least one fluid port may directly fluidly couple to the first portion of the catheter lumen.

The first portion may have a larger inner diameter than the second portion.

At least the tapered tip may comprise a lubricious coating.

The tapered tip may taper continuously.

The drainage catheter assembly may further comprise a 0.018 inch (0.46 millimeter) diameter guide wire that can be slidably received through the first and second portion.

The guide wire may comprise a guide wire distal end that is curled, soft, shapeable, or flexible, and made from a shape-memory material.

The drainage catheter assembly may further comprise an imaging structure provided on at least one of the catheter or stiffening rod, with the imaging structure having at least one imaging modality.

The at least one imaging modality may be: computed tomography, fluoroscopy, magnetic resonance imaging, mammography, x-ray, positron emission tomography, or ultrasound.

The imaging structure may comprise a series of gradations.

The series of gradations may comprise a distance scale.

### BRIEF DESCRIPTION OF THE DRAWINGS

Embodiments are described, by way of example only, with reference to the accompanying drawings, in which:
FIG. 1 is a cross section of a drainage catheter assembly suitable for insertion over a guide wire and including a drainage catheter with a slidably-removable hollow stiffening rod;
FIG. 2 is a schematic illustration of an introducer kit, which can be used in placing the drainage catheter of FIG. 1;
FIG. 3 is a flow chart illustrating a method for draining a site in a tissue mass;
FIGS. 4-8 illustrate the steps of a method of using an insertion kit to locate the guide wire in a tissue mass needing draining and locating the drainage catheter to drain the tissue mass;
FIG. 9 is a schematic illustration of a drainage catheter for the drainage catheter assembly of FIG. 1 according to another aspect of the disclosure herein;
FIG. 10 is a variation of the drainage catheter of FIG. 9 according to yet another aspect of the disclosure herein;
FIG. 11 is the schematic of FIG. 10 with an alternate guide wire provided within the drainage catheter;and
FIG. 12 is the schematic of FIG. 11 with yet another drainage catheter provided over the alternate guide wire.

### DETAILED DESCRIPTION

A drainage catheter is a hollow tube-like structure inserted into a tissue mass at a location that has accumulated fluid in order to remove the fluid build-up. Typically, drainage catheters slide over a pre-positioned guide wire to ensure the drainage catheter is placed in the desired location. Drainage catheters, by way of non-limiting example, can be placed in multiple locations and used in the extraction of fluids from, but not limited to, obstructed kidneys (hydronephrosis), blocked bile ducts (obstructive jaundice), an obstructed gallbladder (cholecystitis), abscesses, cysts, pseudocysts, pneumothoraces, pleural effusions, hematomas, bilomas, or urinomas.

A dilator can have a similar hollow tube-like structure to a drainage catheter and can also slide over a guide wire for placement. A dilator is used to enlarge the access through the tissue mass to the location that has accumulated fluid. The dilator is typically used to create the enlarged access, making for easier and less resistant sliding placement of the drainage catheter. Typically, dilators are made of a rigid material, unlike the flexible material of drainage catheters, based on the primary use of dilators, which is sliding insertion through a tissue mass to enlarge the access passage. Under such an intended use, dilators must be capable of resisting buckling while piercing the tissue mass. While dilators can be used for short-term drainage, they are typically replaced with a catheter.

A sheath dilator is a dilator inside a sleeve known as the sheath. There is often a coaxial connection that secures the dilator within the sheath. A sheath dilator also slides over a guide wire for placement in the body, however, the dilator can be removed from the body and the sheath leaving access to the body through the sheath. Catheters, wires, and other medical tools can then be placed in the body to the proper location through the sheath.

Current methods of placing a drainage catheter within a tissue mass require exchanging a first, smaller diameter (0.018 inch, 0.46 millimeters), guide wire for a second, larger diameter (0.035 inch, 0.90 millimeters), guide wire. There are multiple reasons for the exchanging of the guide wires. The smaller 21 gauge needle through which the 0.018 inch (0.46 millimeters) guide wire is advanced is easier to accurately place and causes less trauma to the tissue, leading to its preferred use in initially locating the target site in the tissue mass. The larger guide wire is stiffer and less resistant to buckling during the sliding of any of the catheter, dilator or sheath dilator over the guide wire to the desired location. Also, for historical reasons, the lumen of catheters, dilators, and sheath dilators have been sized for the larger guide wire. For functional reasons, the larger lumen is preferred in catheters to obtain a desired fluid flow rate. A lumen sized for the smaller guide wire typically has undesirably slow flow rate.

If the larger lumen is slid over the smaller guide wire, a relatively large annular gap is formed between the small guide wire and the lumen. The annular gap is sufficiently large, especially between the 0.018 inch (0.46 millimeters) guide wire and 0.035 inch (0.90 millimeters) lumen, that tissue can enter the annular gap, which will negatively impact the sliding of the catheter, dilator, or sheath dilator. The negative impact on sliding can result in sufficient resistance to prevent smooth sliding and accurate placement or can be great enough to completely prevent sliding.

The kit and/or drainage catheter that is the subject of this description provides for a drainage catheter that can be slid over the smaller guide wire and still retain the desired flow rate of the traditional larger lumen catheter, while also negating the need to exchange guide wires, which simplifies the installation of the catheter and reduces the time to install. Such a drainage catheter and method are a substantial improvement over prior drainage catheters and methods of placement.

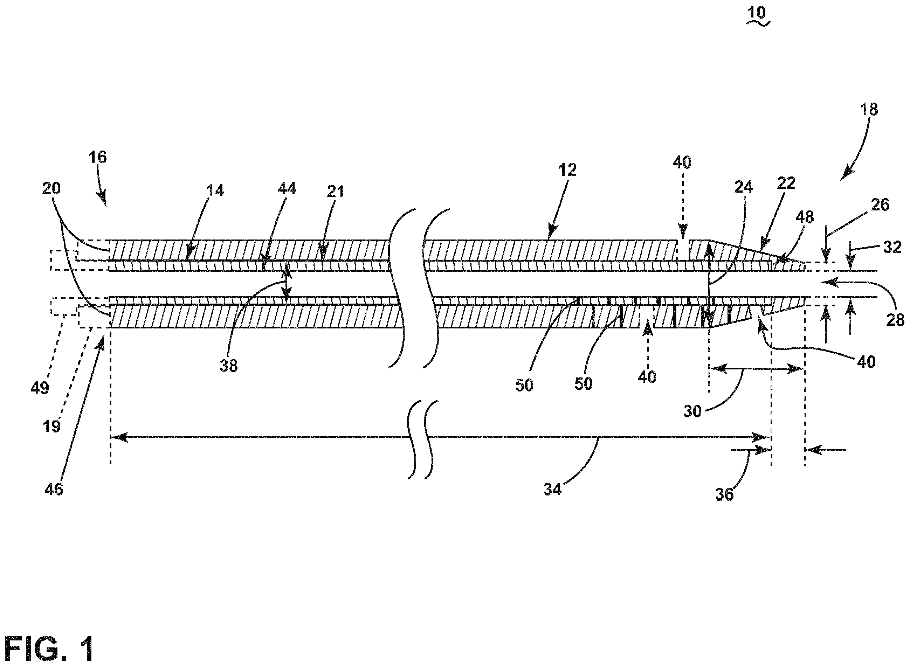

Turning to FIG. 1, we can examine such a drainage catheter, with FIG. 1 showing a cross section of a drainage catheter assembly 10 that includes a hollow catheter 12 and a hollow stiffening rod 14 suitable for insertion over a guide wire 60. The catheter 12 includes a proximal end 16 and a distal end 18 relative to the user. The proximal end 16 can include various connectors 19, such as, but not limited to, Luer lock type connectors.

The catheter 12 can be flexible and include a hollow body 20 that defines a catheter lumen 21. The hollow body 20 extends from the proximal end 16 to the distal end 18 and terminates with a tapered tip 22. The tapered tip 22 begins at a first outside diameter 24 and decreases to a second outside diameter 26. At the second outside diameter 26, the catheter lumen 21 intersects the tapered tip 22 to form a tip outlet 28.

By way of non-limiting example, the tapered tip 22 can taper continuously; that is, the change in diameter per a length 30 or the slope from the first outside diameter 24 to the second outside diameter 26 can be constant. Alternatively, the tapered tip 22 can taper at varying slopes or have a non-continuous taper, such as a multi-stepped taper.

The tapered tip 22 can include a lubricious coating, a hydrophilic lubricious coating, or a hydrophilic coating. The lubricious coating can decrease the friction between the catheter 12 and body tissue, which is especially advantageous as it can prevent buckling of the wire and help ensure successful advancement of the catheter 12 through high resistance tissues. It is contemplated that any portion of the catheter 12 can include a lubricious coating, a hydrophilic lubricious coating, or a hydrophilic coating.

The catheter lumen 21 can include a first portion 34 and a second portion 36. The first portion 34 can have a first portion ID 38 that is greater than an ID 32 of the second portion 36. As illustrated, the first portion 34 of the catheter lumen 21 can extend into a portion of the tapered tip 22.

The second portion 36 of the catheter lumen 21 includes at least the tip outlet 28 and shares magnitude inner diameter as the ID 32 of the tip outlet 28. The second portion 36 can include part of or all of the tapered tip 22. Optionally, the second portion 36 can include the tip outlet 28 and any portion of the catheter 12 that does not receive the stiffening rod 14.

The stiffening rod 14 can be located in the first portion 34 of the catheter lumen 21. The stiffening rod 14 can define a stiffening rod lumen 44 that extends from a rod proximal end 46 to a rod distal end 48. The rod proximal end 46 can include various connectors 49, such as, but not limited to, Luer lock type connectors. The various connectors 19, 49 can secure the stiffening rod 14 within the catheter 12.

At least one fluid port 40 can be located in the hollow body 20 of the catheter 12 at the distal end 18. The at least one fluid port 40 is illustrated, by way of non-limiting example, as a channel extending from the catheter lumen 21 through the hollow body 20. It is contemplated that the at least one fluid port 40 can include multiple fluid ports 40. Optionally, the at least one fluid port 40 directly fluidly couples to the first portion 34 or the second portion 36 of the drainage catheter assembly 10.

The location of the at least one fluid port 40 can be used to increase flow rate into the catheter lumen 21. It is contemplated that the at least one fluid port 40 couples to the first portion 34 of the catheter lumen 21 to improve flow rate due to the first portion ID 38 being greater than the ID 32 of the second portion 36 and the ID 32 of the tip outlet 28. It is further contemplated that a number of fluid ports 40 can be determined to compensate for the area difference between the tip outlet 28 and the first portion 34 cross-sectional area.

An imaging structure 50 can be located on the catheter 12 or the stiffening rod 14. The imaging structure 50 has at least one imaging modality. Modalities can include, but are not limited to, computed tomography, fluoroscopy, magnetic resonance imaging, mammography, x-ray, position emission tomography, or ultrasound. As illustrated, by way of non-limiting example, the imaging structure 50 can be a set of imaging structures 50 visible during selected imaging modality. The set of imaging structures 50 can appear as a set of gradations. Alternatively, the imaging structure 50 can be a continuous structure that includes a series of gradations. The series of gradations can include a distance scale. By way of non-limiting example, the series of gradations can indicate 2 millimeters between each mark on the catheter 12 or stiffening rod 14.

Additionally or alternatively, the series of gradations or imaging structure 50 can be gradations located at known positions of the catheter assembly 10. Known positions can include, but are not limited to, the distal end 18 of the tapered tip 22, the rod distal end 48 of the stiffening rod 14, or the fluid ports 40.

A dimensional review of the catheter 12 and stiffening rod 14 will be helpful in understanding the utility of the catheter assembly 10. It will be further helpful to understand some terms as part of that review. As used herein, the term "marginally larger" is used to indicate a size difference that provides a "sliding clearance fit." In engineering terms, the "fit" is the clearance between two mating parts. The size of the clearance determines whether the parts can move independently from each other, are temporarily joined, or are permanently joined. The term "sliding fit clearance" can be defined as at least the smallest or minimal clearance between the two mating parts in which the parts can be easily assembled and both parts are still capable of turning or sliding freely.

In a dimension example relevant to this description, a first object that has an OD of 0.018 inch (0.46 millimeters) would maintain a sliding fit clearance when slidably received inside a second object with an ID that is marginally larger than 0.018 inch (0.46 millimeters). Minimizing any gap between the first and second objects can prevent or minimize tissue or other materials from enter the gap. Material in the gap can interfere with the free sliding of the first object within the second object.

The first outside diameter 24 of the catheter 12 can range from 4 French (0.0525 inches, 1.33 millimeters) to 10.5 French (0.138 inches, 3.50 millimeters). Alternatively, the first outside diameter 24 of the catheter 12 can be larger than 10.5 French. The ID 32 of the second portion 36 of the catheter lumen 21 and the tip outlet 28 are marginally larger than 0.018 inch (0.46 millimeters). The first portion ID 38 of the catheter lumen 21 is marginally larger than 0.035 inches (0.90 millimeters).

The stiffening rod 14 can have an outside diameter of approximately 0.035 inches (0.90 millimeters). The inner diameter of the stiffening rod lumen 44 can be the ID 32 which is marginally larger than 0.018 inches (0.46 millimeters).

The dimensional relationships described allow the stiffening rod 14 to slide in and out of the catheter lumen 21. When the stiffening rod 14 is removed from the catheter lumen 21 the at least one fluid port 40 connects to the first portion 34 of the catheter lumen 21.

The tip outlet 28 and second portion 36 of the catheter 12 have the ID 32 of marginally larger than 0.018 inches (0.46 millimeters). The ID 32 is smaller than the tip outlet of prior catheters that are 6 French or greater. The ID 32 is desired, as it is similar to an introduction needle.

The catheter 12 includes the first portion 34 with the first portion ID 38 that is marginally larger than 0.035 inches (0.90 millimeters). The first portion ID 38 is comparable to prior catheters; however, the prior catheters often include a tip outlet with an inner diameter of marginally larger than 0.035 inches (0.90 millimeters) to ensure fluid flow. While fluid can flow through the smaller tip outlet 28 into the catheter lumen 21, the connecting of the at least one fluid port 40 to the first portion 34 of the catheter lumen 21 establishes a greater fluid flow path. Having the first portion 34, the second portion 36, and the at least one flow port 40 allows the catheter 12 have both the smaller tip outlet 28 and excellent fluid flow.

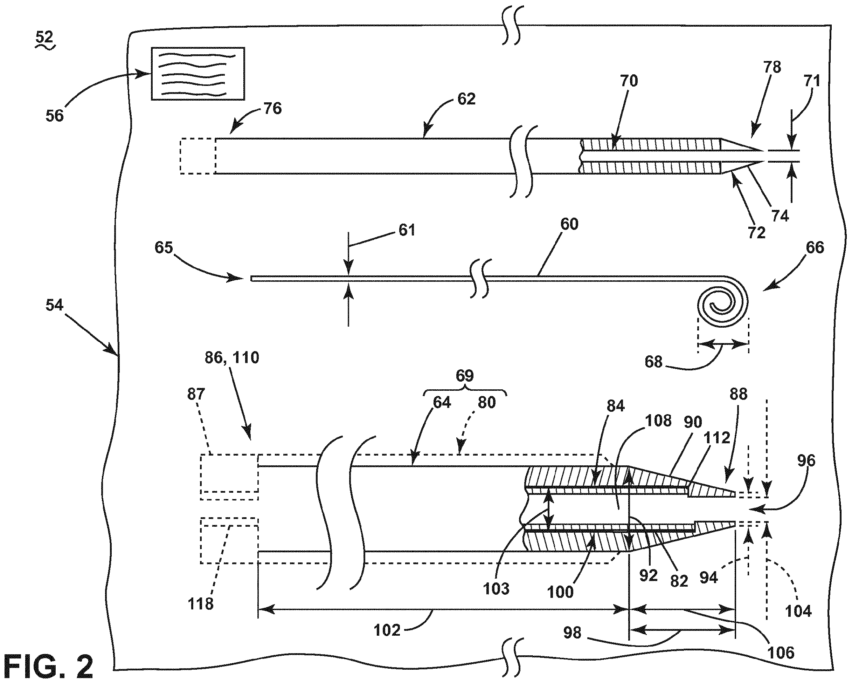

FIG. 2 illustrates a guide wire kit 52 suitable for use with the catheter assembly 10 of FIG. 1. The guide wire kit 52 includes a package 54 having a label 56 with suitable instructions/information regarding the guide wire kit 52, and at least a guide wire 60 and an introducer needle 62, with a dilator 64. Optionally, the dilator 64 can couple to a sheath 80 to form a sheath dilator 69. It is further contemplated that the catheter assembly 10 can be optionally included in the guide wire kit 52.

The guide wire 60, having a guide wire OD 61, can include a guide wire proximal end 65 and a guide wire distal end 66. A curled portion 68 of the guide wire 60 can be located at the guide wire distal end 66. At least a part of the curled portion 68 can be soft, flexible, shapeable, or curled; made of a shape-memory material or other alloy. One non-limiting example of a shape memory material or alloy is nitinol. The guide wire 60, having the guide wire OD 61, can include a guide wire proximal end 65 and a guide wire distal end 66. The guide wire 61 can be made from a lubricious shape memory material that resists kinking. A curled portion 68 of the guide wire 60 can be located at the guide wire distal end 66. The curled portion 68 can be flexible or made of a shape-memory material or alloy. One non-limiting example of a shape memory material or alloy is nitinol.

Optionally, additional imaging structures can be included on the guide wire 60 to detection of one or more locations on the guide wire 60 while viewing with one or more image modalities. Additionally, the guide wire 60 can include a lubricious coating. It is contemplated that the guide wire OD 61 is 0.018 inch (0.46 millimeters) or less.

A needle lumen 70 can be defined by the hollow portion of the needle 62. By way of non-limiting example, the needle 62 can be between 19 gauge and 21 gauge. Typically 19 gauge to 21 gauge needles have a needle lumen ID 71 that is between 0.027 inches (0.686 millimeters) and 0.0203 inches 0.514 millimeters (0.514 millimeters) respectively. Alternatively, the needle 62 can be any gauge as long as the needle lumen ID 71 is marginally larger than a guide wire OD 61 of the guide wire 60 selected by the user.

The needle 62 has a needle proximal end 78 and a needle distal end 74. An insertion tip 72 at the needle distal end 74 can include a sharpened tip 76. The sharpened tip 76 can be a beveled tip. Alternatively, by way of non-limiting examples, the sharpened tip 76 can be a single bevel, multi-bevel with multi-facet, Tuohy, Franseen, Mengini, back cut bevel, or Dos Santos style needle points. Optionally, the tips can be designed for greater viability under ultrasound guidance.

The dilator 64 can include a hollow body or tubular body 84 that defines a dilator lumen 100. The tubular body 84 extends from a dilator proximal end 86 to a dilator distal end 88 and terminates with a dilator tapered tip 90. The dilator proximal end 86 can include various connectors 87, such as, but not limited to, Luer lock type connectors.

The tapered tip 22 begins at a first outside diameter 24 and decreases to a second outside diameter 26. At the second outside diameter 26, the catheter lumen 21 intersects the tapered tip 22 to form a tip outlet 28. The dilator tapered tip 90 begins at a first dilator outside diameter 92 and decreases to a second dilator outside diameter 94. At the second dilator outside diameter 94, the dilator tapered tip 90 includes a dilator tip outlet 96.

By way of non-limiting example, the dilator tapered tip 90 can taper continuously; that is, the change in diameter per a dilator length 98 or the slope from the first dilator outside diameter 92 to the second dilator outside diameter 94 can be constant. Alternatively, the dilator tapered tip 90 can taper at varying slopes or have a non-continuous taper, such as a multi-stepped taper.

The dilator tapered tip 90 can include a lubricious coating, a hydrophilic lubricious coating, or a hydrophilic coating. The lubricious coating can decrease the friction between the dilator 64 and body tissue. It is contemplated that any portion of the dilator 64 or the sheath dilator 69 can include a lubricious coating, a hydrophilic lubricious coating, or a hydrophilic coating. It is also contemplated that the tubular body 84 can include at least one fluid port (not shown) at the distal end 88.

The dilator lumen 100 can include a first dilator portion 102 and a second dilator portion 106. The first dilator portion 102 can have a first dilator portion ID 103 that is greater than a second dilator portion ID 104 of the second dilator portion 106.

Optionally, a dilator stiffening rod 82 can be located in the first dilator portion 102 of the dilator lumen 100. The dilator stiffening rod 82 can define a dilator stiffening rod lumen 108 that extends from a dilator rod proximal end 110 to a dilator rod distal end 112. The dilator rod proximal end 110 can include various connectors 118, such as, but not limited to, Luer lock type connectors.

Optionally, imaging structures can be located on the needle 62, dilator 64, sheath 80, or the dilator stiffening rod 82. The imaging structure has at least one imaging modality. Modalities can include, but are not limited to, computed tomography, fluoroscopy, magnetic resonance imaging, mammography, x-ray, position emission tomography, or ultrasound. As illustrated, by way of non-limiting example, the imaging structure can be a set of imaging structures visible during selected imaging modality. The set of imaging structures can appear as a set of gradations. Alternatively, the imaging structure can be a continuous structure that includes a series of gradations. The series of gradations can include a distance scale.

Additionally or alternatively, the series of gradations or imaging structure can be gradations located at known positions of the dilator 64 or sheath dilator 69. Known positions can include, but are not limited to, the dilator distal end 88 of the dilator tapered tip 90, the dilator rod proximal end 110 of the dilator stiffening rod 82, or portions of the sheath 80.

The dilator 64 can have a size from 4 French to 10.5 French where the first dilator outside diameter 92 of the dilator 64 can range from 1.333 millimeters (0.0525 inches) to 3.500 millimeters (0.138 inches) respectively.

The second dilator portion ID 104 of the dilator lumen 100 and the dilator tip outlet 96 are marginally larger than 0.455 millimeters (0.018 inches). The first dilator portion ID 103 of the dilator lumen 100 is marginally larger than 0.899 millimeters (0.035 inches).

The dilator stiffening rod 82 can have an outside diameter of approximately 0.899 millimeters (0.035 inches). The dilator stiffening rod 82 can define a dilator stiffening rod lumen 108. The dilator stiffening rod lumen 108 can be the same dimensions as the second dilator portion ID which is marginally larger than 0.455 millimeters (0.018 inches).

The first dilator portion 102 of the dilator lumen 100 can slidably receive the dilator stiffening rod 82. Once slidably received, the various connectors 87, 118 can secure the dilator stiffening rod 82 within the dilator 64. As illustrated, the first dilator portion 102 of the dilator lumen 100 can extend into a portion of the dilator tapered tip 90.

The second dilator portion 106 of the dilator lumen 100 includes at least the dilator tip outlet 96 and shares the same ID 104 as the dilator tip outlet 96. The second dilator portion 106 can include part of or all of the dilator tapered tip 90. Optionally, the second dilator portion 106 can include the dilator tip outlet 96 and any portion of the dilator 64 that does not receive the dilator stiffening rod 82.

The sheath 80 can slidably couple to the dilator 64. It is contemplated that the inner diameter of the sheath 80 is marginally larger than the first dilator outside diameter 92 of the dilator 64. The sheath dilator 69 can have a size between 4 French and 10.5 French.

The dilator tip outlet 96, the second dilator portion 106, and the dilator stiffening rod 82 have the second dilator portion ID that is marginally larger than the guide wire OD 61.

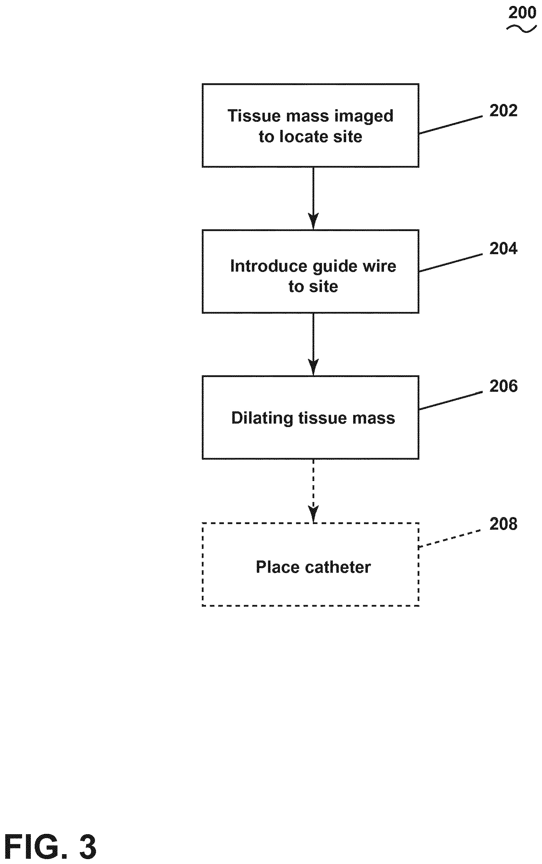

FIG. 3 illustrates a general overview of a method 200, by which the guide wire kit of FIG. 2 and optionally the catheter assembly 10 of FIG. 1 can be used to drain a site in a tissue mass. At 202, a tissue mass is imaged to locate a site. At 204, while imaging, the guide wire 60 is percutaneously and non-intravenously introduced into the site using the needle 62. The needle 62 is removed and at 206, the tissue mass is dilated using the dilator 64 or the sheath dilator 69 that slidably fit around the guide wire 60. Optionally, at 208, the drainage catheter assembly 10 is placed at the site. Further, optionally, the catheter assembly 10 can be used in place of the dilator 64 at 206, such that the catheter assembly performs the dilation function. This is possible because the stiffening rod 14 provides the otherwise flexible catheter 12 with sufficient stiffness.

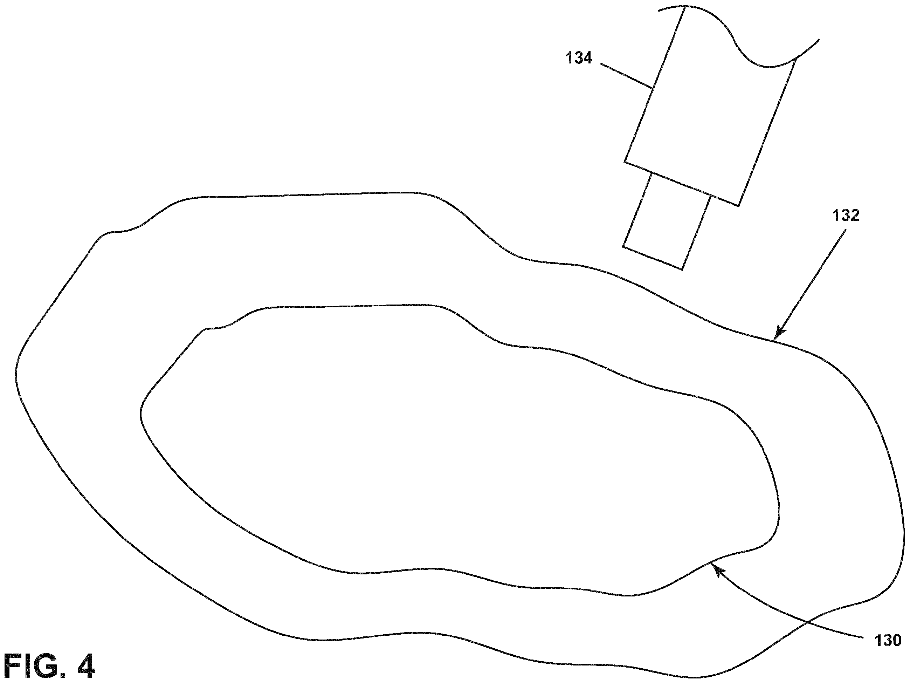

FIGS. 4-8 further schematically illustrate the method 200 in view of the relative position and arrangement of the catheter assembly 10 and the guide wire kit 52. Beginning with FIG. 4, which illustrates 202 of the method 200, a tissue mass 132 is imaged to locate the site 130. The site 130 is illustrated, by way of non-liming example, as an abscess. An imaging device 134 can be used to perform the imaging needed to locate the site 130 in the tissue mass 132. The imaging device 134 can be a component used in imaging modalities such as, but not limited to, computed tomography, fluoroscopy, magnetic resonance imaging, mammography, x-ray, position emission tomography, or ultrasound.

After locating the site 130 at 202 and while still imaging, the needle 62 is placed through the tissue mass 132 so that the needle distal end 74 penetrates the site 130. The imaging device 134 can be used to ensure proper placement of the introduction needle 62. Optionally, imaging structures can be placed on or in the introduction needle to increase visibility during placement into the site 130.

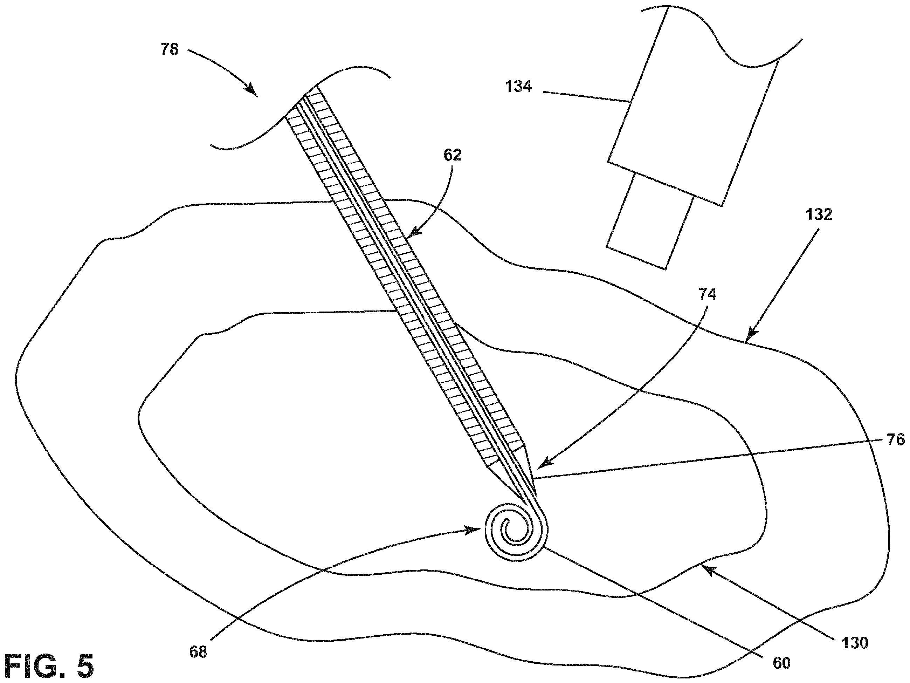

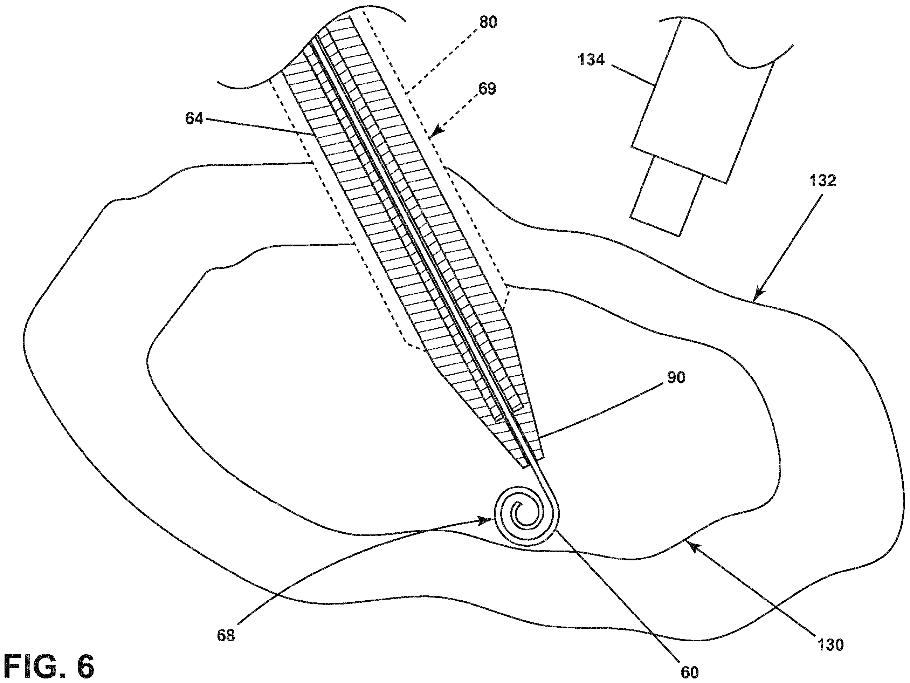

Once the needle distal end 74 of the needle 62 is properly placed in the site 130, the guide wire 60 is percutaneously and in an extra vascular manner introduced into the site 130 through the needle lumen 70. As seen in FIG. 5, at 204, the curled portion 68 of the guide wire 60 can extend into the site 130 past the needle distal end 74. Optionally, imaging structures can be placed on or in the guide wire 60 to increase visibility by the imaging device 134 during placement into the site 130. At 206, once the guide wire 60 is properly placed in the site 130, the needle 62 can be slidably removed from the site 130 and the tissue mass 132 and the tissue mass is dilated by sliding the dilator 64 over the guide wire 60, so the guide wire 60 is received in the dilator lumen 100 via the dilator tip outlet 96. The dilator 64, which optionally be a sheath dilator 69, slides along the guide wire 60 through the tissue mass 132 and into the site 130, which is illustrated in FIG 6. This dilates access to the site 130 in the tissue mass 132 to at least the size of the first dilator outside diameter 92.

According to one variation, the dilator 64 can be removed from the site 130 and the tissue mass 132; leaving the guide wire 60 in place. According to another variation, when the sheath dilator 69 is used, the dilator 64 can be removed leaving the guide wire 60 and the sheath 80.

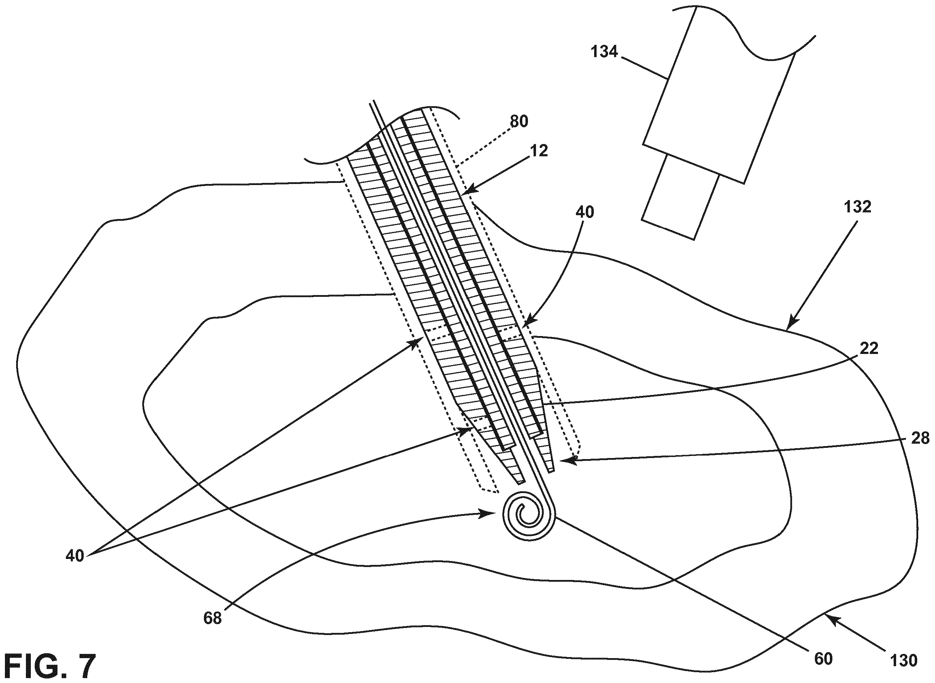

In the variant where the dilator 64 is removed, the catheter 12 can be slid over the guide wire 60 to place the catheter 12 into the abscess to drain the abscess. To place the catheter 12, the tip outlet 28 of the catheter 12 slidably receives the guide wire 60 at the guide wire proximal end 65. The catheter 12 is then advanced over the guide wire 60, such that the guide wire 60 slides through the second portion 36. The stiffening rod 14 contained in the first portion 34 of the catheter 12 slidably fits around the guide wire 60 at the guide wire proximal end 65, so that the guide wire 60 slides into the stiffening rod lumen 44 from the second portion 36. It is contemplated that the stiffening rod lumen 44 is marginally larger than the diameter of the guide wire 60. The catheter 12 is slid along the guide wire 60 through the tissue mass 132 and into the site 130, which is illustrated in FIG. 7. The drainage catheter assembly 10 can be placed while imaging using the imaging device 134. While the illustration reveals a gap between the stiffening rod lumen 44 and the guide wire 60; it is only for clarity between the components.

In the variation where the sheath 80 is left, the catheter 12 can be placed in the tissue mass as just described, except that the catheter 12 slides inside the sheath 80, while sliding over the guide wire 60. Sliding the catheter 12 through the sheath 80 provides much less resistance than sliding the catheter 12 through the tissue mass, even a dilated tissue mass. Optionally, to further reduce sliding friction, the lubricious coating can decrease the friction between the catheter 12 and the tissue mass 132 or sheath 80.

It is further contemplated that the imaging structures 50 can be placed on or in the catheter 12 or the stiffening rod 14 to increase visibility by the imaging device 134 during placement into the site 130. By way of non-limiting example, how far to slide the drainage catheter assembly 10 into the tissue mass 132 can be determined by the imaging structure 50. The sliding of the drainage catheter assembly 10 into the tissue mass 132 can cease once the imaging structure 50, as viewed by the imaging device 134, reaches a desired location relative to the site 130.

The distal end 18 of the catheter 12 can include a curled portion 120. The curled portion 120 contains the at least one fluid port 40. The stiffening rod 14 can be used to keep the curled portion 120 of the catheter 12 straight when received in the catheter lumen 21.

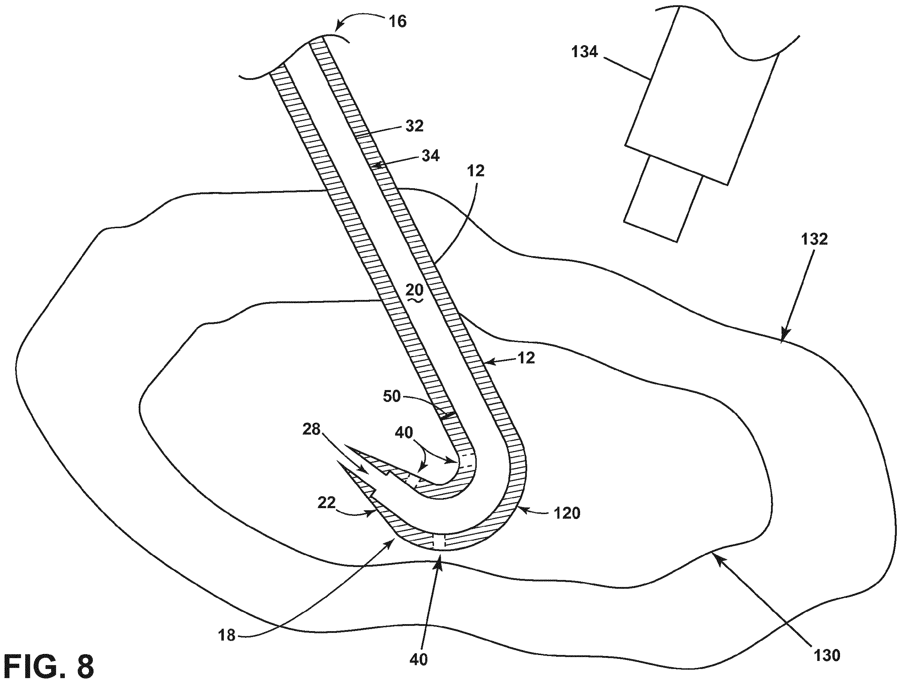

Once the drainage catheter assembly 10 is placed at the site 130, the stiffening rod 14 is removed, allowing the curled portion 120 to curve, which helps to anchor the catheter 12 at the site 130. The guide wire 60 can also be removed before, with, or after the stiffening rod 14 is removed. Optionally, if a sheath 80 is used for placement of the catheter 12, it can also be removed. As illustrated in FIG. 8, when the stiffening rod 14 is removed from the catheter lumen 21 the at least one fluid port 40 connects to the first portion 34 of the catheter lumen 21.

To remove the catheter 12, the wire 60 with or without the stiffening rod is reinserted into the catheter lumen 21 to straighten or partially straighten the curled tip and reform or partially reform the catheter assembly 10, which is then withdrawn from the tissue mass.

The catheter assembly 10 as described, with the dual size lumen and side ports, provides for the placement of the catheter assembly 10 with a smaller guide wire while still providing the flow rate of the traditional larger size lumen, while negating the need to swap guide wires, which simplifies and shortens the time for placing the catheter 12. This particularly important for procedures using ionizing radiation where the dose to the patient and operator can be decreased using by using the catheter assembly 10 or the guide wire kit 52 disclosed herein.

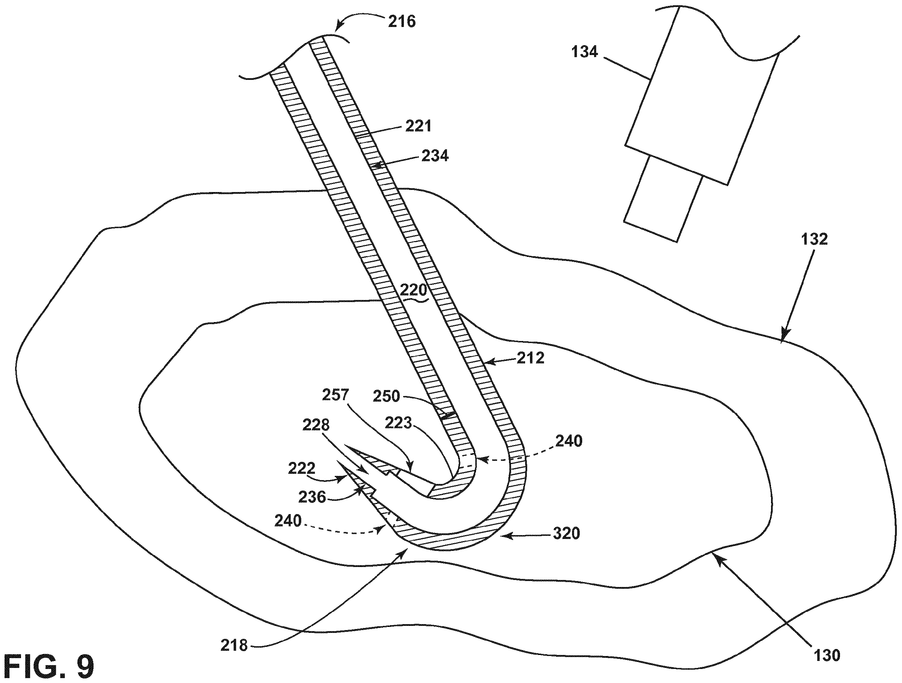

Optionally, at 208 the placement of a catheter 212 can include a catheter exchange in which the catheter 212 is exchanged for larger catheter. FIGS. 9-10 illustrate a catheter 212 located in the site 130 that is substantially similar to the catheter 12. Therefore, like parts will be identified with like numerals increased by 200, with it being understood that the description of the like parts of the catheter 12 applies to the catheter 212 unless otherwise noted.

The catheter 212 is structurally similar to the catheter 12 except that the catheter 212 includes an exit port 257. The exit port 257 can be located at the distal end 218 of the catheter 212 adjacent to or adjoining a tapered tip 222. It is contemplated that the exit port 257 is located in a curled portion 320 of the catheter 212.

The exit port 257 is an opening larger than at least one fluid port 240 with sufficient size to receive a guide wire as large as a hollow body 220. By way of non-limiting example, larger guide wires can be on the order of 0.035 inches (0.90 millimeters) or 0.038 inches (0.97 millimeters) in diameter. The exit port 257 allows for a guide wire larger than a tip outlet 228 to be used to remove the catheter 212.

It is contemplated that the catheter 212 can be used in the drainage catheter assembly 10 in place of the catheter 12 or the dilator 64. For example, the catheter 212 can be a part of the drainage catheter assembly 10 and located in the site 130 using the guide wire kit 52, the stiffening rod 14, and optionally the sheath 80. The catheter 212 illustrated in FIGS. 9-10 is located in the site 130 after the stiffening rod 14 and the guide wire 60 have been removed.

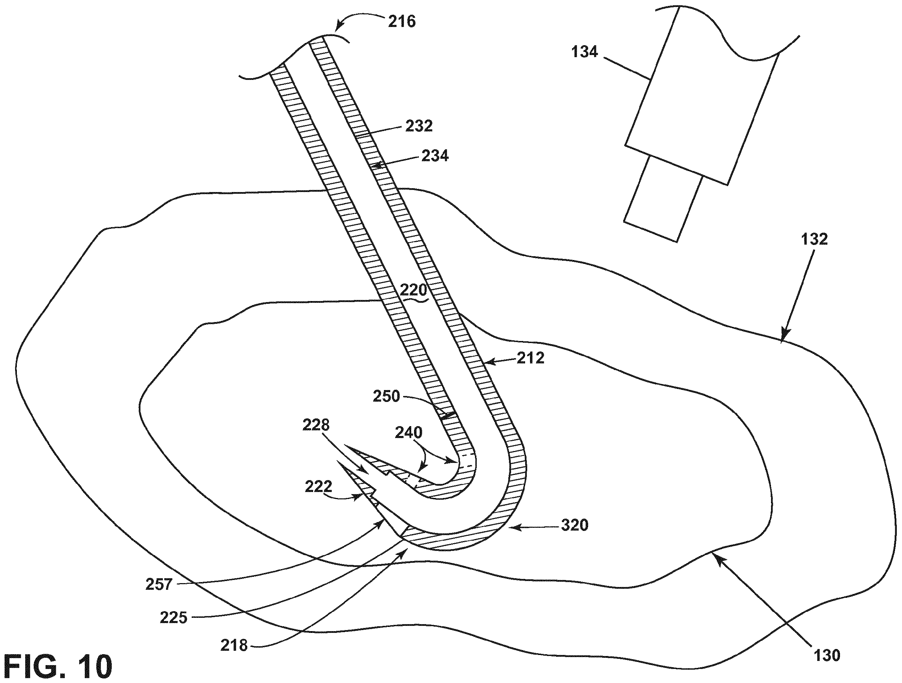

It is by way of non-limiting example that the exit port 257 is illustrated in FIG. 9 at an inner curved portion 223 of the curled portion 320. FIG. 10 illustrates the catheter 212 in another non-limiting example in which the exit port 257 is located in an outer curved portion 225 of the curled portion 320. It is contemplated that the catheter 212 can include any number of exit ports 257 located adjacent to or adjoining a tapered tip 222.

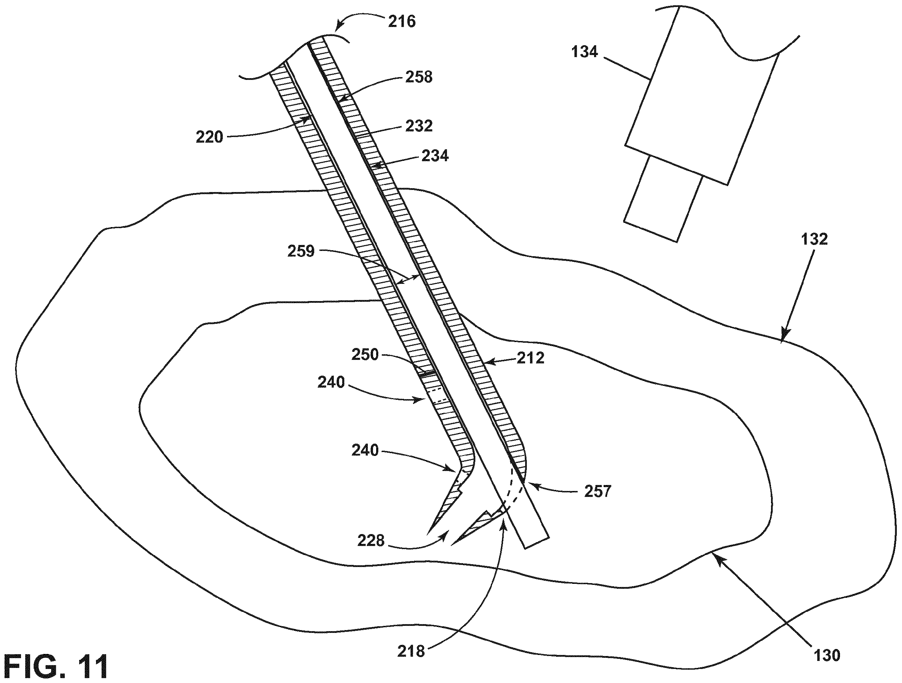

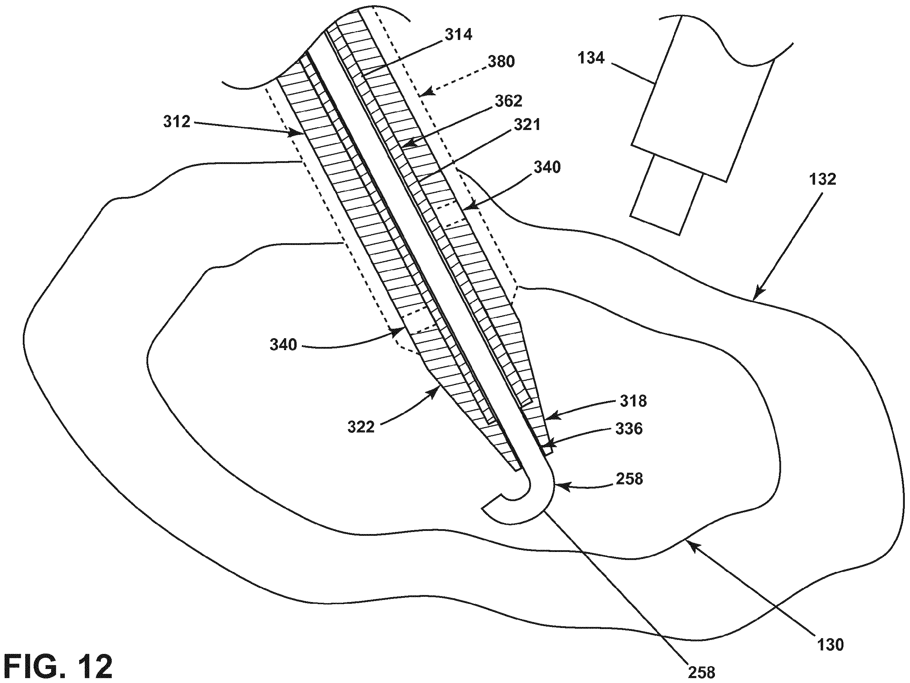

FIGS. 11-12 illustrate the catheter exchange from the catheter 212 in FIG. 10 to a larger catheter 312 in FIG. 12. The catheter exchange can optionally include a guide wire swap using a second guide wire 258. It is contemplated that the second guide wire OD 259 is 0.035 inches (0.90 millimeters) or greater. Optionally, additional imaging structures can be included on the second guide wire 258 to detect one or more locations on the second guide wire 258 while viewing with one or more image modalities. Additionally, the second guide wire 258 can include a lubricious coating.

The larger catheter 312 is structurally similar to the catheter 12 except that the larger catheter 312 includes at least a larger hollow body 362 defining a larger catheter lumen 321. Therefore, like parts will be identified with like numerals increased by 300, with it being understood that the description of the like parts of the catheter 12 applies to the catheter 312 unless otherwise noted.

The catheter exchange can begin once the catheter 212 is located in the site 130 with the guide wire 60 and stiffening rod 14 removed. A guide wire distal end 266 of the second guide wire 258 can enter a proximal end 216 of the catheter 212. The second guide wire 258 can then pass through the hollow body 220 and exit the catheter 212 through the exit port 257. The catheter 212 can be removed once the second guide wire 258 is located in the site 130.

The larger catheter 312 can be placed into the site 130 by sliding the larger guide wire proximal end 365 through the tip outlet 328 and into the hollow body 362. It is contemplated that the larger catheter 312 can be a larger dilator with a sheath 380. It is further contemplated that the catheter exchange can be completed using the guide wire 60.

While the invention is described in the customary guide wire sizes of 0.018", 0.035" and 0.038" and the corresponding catheter, dilator, needles, with their corresponding appropriately sized lumens, the invention is not limited to these two specific size guide wires. That said, as these two size guide wires are industry standards, and the prior catheters, dilators, needles, have been sized accordingly, the invention will find great and immediate applicability for these size guide wires.

Throughout this document, reference to sizes are in terms of their common or historical scale, which include: gauge, inches, and French. For the ease of comparison, the corresponding dimension has been converted to millimeters and placed in parentheses. The conversion value is provided to two decimal places, but this is for convenience only, and no special significance should be ascribed the use of two or three significant digits.

Benefits of the aspects described herein can include a decrease in the number of steps or the amount of time it takes to provide a larger catheter to a predetermined site by obviating the need for upsizing the 0.035 inch (0.90 millimeters) or 0.038 inch (0.97 millimeters) guide wire.

Additionally, a decrease in the amount of time to place a catheter at a predetermined site can result in a reduction of the ionizing radiation used when placing the catheter.

Further, the aspects described herein, utilize the traditional 21- or 22-gauge needle with the decrease in the number of steps or the amount of time it takes. The 21- or 22-gauge needle is viewed as having an enhanced safety profile due to its size. For example, the 21- or 22-gauge needle can provide a greater precision in placing the wire and subsequently the catheter into a smaller target site as opposed to larger needles.

Additionally, the smaller needle diameter is less injurious to the tissues with a lower bleeding risk compared to larger needles. For example, the bowel can generally be inadvertently safely traversed with a 21- or 22-gauge needle while larger needles may lead to bowel perforation with peritonitis and or abscess formation necessitating a secondary procedure or surgery. The chance of clinically significantly bleeding is reduced by utilizing a 21 or 22-gauge needle as opposed to an18-gauge or larger needle. For example, the smaller needle not only allows one to more safely navigate near a blood vessel with greater precision, but also if that blood vessel is inadvertently traversed with the needle the likelihood of bleeding is reduced.

The smaller 0.018 wire placed through the smaller needle utilized in the kit described herein also allow for the selection of smaller targets since the larger 0.035 or 0.038 wire cannot readily coil within very small cavities and therefore can buckle out of the cavity or target site.

Additionally, eliminating a 035-or.038-wire exchange in order to place the catheter not only reduces the time to complete he procedure but also but also improves the efficacy and safety of the procedure. The traditional 0.035 or 0.038 wire exchange when performed without fluoroscopy or direct visualization (computerized tomography or ultrasound guided procedures) allows for the possibility of losing wire access to the target requiring the operator to start the procedure over.

To the extent not already described, the different features and structures of the various embodiments can be used in combination, or in substitution with each other as desired. That one feature is not illustrated in all of the embodiments is not meant to be construed that it cannot be so illustrated, but is done for brevity of description. Thus, the various features of the different embodiments can be mixed and matched as desired to form new embodiments, whether or not the new embodiments are expressly described. All combinations or permutations of features described herein are covered by this disclosure.

This written description uses examples to disclose the invention, including the best mode, and also to enable any person skilled in the art to practice the invention, including making and using any devices or systems and performing any incorporated methods. The patentable scope of the invention is defined by the claims, and can include other examples that occur to those skilled in the art. Such other examples are intended to be within the scope of the claims if they have structural elements that do not differ from the literal language of the claims, or if they include equivalent structural elements with insubstantial differences from the literal languages of the claims.

Further aspects of the invention are provided by the subject matter of the following clauses:
1. A drainage catheter assembly comprising a catheter comprising a flexible, hollow body terminating in a distal end comprising a tapered tip, and the hollow body defining a catheter lumen with a tip outlet in the tapered tip, a hollow stiffening rod slidably received within the catheter lumen and defining a stiffening rod lumen marginally greater than 0.455 millimeters (0.018 inches), the catheter lumen having a first portion sized to slidably receive the stiffening rod and a second portion, at least including the tip outlet, and marginally greater than 0.455 millimeters (0.018 inches), and at least one fluid port located in the hollow body at the distal end.
2. The drainage catheter assembly of clause 1 wherein the at least the distal end comprises a curled portion.
3. The drainage catheter assembly of any preceding clause wherein the curled portion is straightened when the stiffening rod is received within the catheter lumen.
4. The drainage catheter assembly of any preceding clause wherein the at least one fluid port is located in the curled portion.
5. The drainage catheter assembly of any preceding clause wherein the at least one fluid port comprises multiple fluid ports.
6. The drainage catheter assembly of any preceding clause where in the first portion extends into the tapered tip.
7. The drainage catheter assembly of any preceding clause wherein the at least one fluid port directly fluidly couples to the first portion of the catheter lumen.
8. The drainage catheter assembly of any preceding clause wherein the first portion has a larger inner diameter than the second portion.
9. The drainage catheter assembly of any preceding clause where in the first portion extends into the tapered tip.
10. The drainage catheter assembly of any preceding clause wherein the at least one fluid port directly fluidly couples to the first portion of the catheter lumen.
11. The drainage catheter assembly of any preceding clause wherein the first portion has a larger inner diameter than the second portion.
12. The drainage catheter assembly of any preceding clause wherein at least the tapered tip comprises a lubricious coating.
13. The drainage catheter assembly of any preceding clause wherein the tapered tip tapers continuously.
14. The drainage catheter assembly of any preceding clause further comprising a 0.018 inch (0.46 millimeters) diameter guide wire that can be slidably received through the first and second portion.
15. The drainage catheter assembly of any preceding clause wherein the guide wire comprises a guide wire distal end.
16. The drainage catheter assembly of any preceding clause wherein the guide wire distal end is curled, soft, shapeable, or flexible.
17. The drainage catheter assembly of any preceding clause wherein the wire or curled portion is flexible and made from a shape-memory material.
18. The drainage catheter assembly of any preceding clause further comprising an imaging structure provided on at least one of the catheter or stiffening rod, with the imaging structure having at least one imaging modality.
19. The drainage catheter assembly of any preceding clause wherein the at least one imaging modality is: computed tomography, fluoroscopy, magnetic resonance imaging, mammography, x-ray, positron emission tomography, or ultrasound.
20. The drainage catheter assembly of any preceding clause wherein the imaging structure comprises a series of gradations.
21. The drainage catheter assembly of any preceding clause wherein the series of gradations comprises a distance scale.
22. A guide wire insertion kit comprising a guide wire having a diameter of 0.018 inch (0.46 millimeters) or smaller, an introduction needle having an insertion tip and defining a needle lumen greater than the diameter of the guide wire, and a hollow dilator of 4 to 10.5 French terminating in a tapered tip and defining a dilator lumen defining an outlet at the tapered tip, with at least the outlet of the lumen having being marginally larger than the diameter of the guide wire to provide a sliding fit clearance for the guide wire.
23. The kit of any preceding clause wherein the dilator is a sheath dilator.
24. The kit of any preceding clause wherein the sheath dilator is of 4 to 10.5 French.
25. The kit of any preceding clause wherein the dilator can include a dilator stiffening rod slidably received within the dilator lumen and defining a dilator stiffening rod lumen marginally greater than 0.455 millimeters (0.018 inches).
26. The kit of any preceding clause wherein the guide wire comprises a guide wire distal end.
27. The kit of any preceding clause wherein the guide wire distal end comprises a curled portion.
28. The kit of any preceding clause wherein the curled portion is flexible and made from a shape-memory material.
29. The kit of any preceding clause further comprising an imaging structure provided on at least one of the dilator or the dilator stiffening rod, with the imaging structure having at least one imaging modality.
30. The kit of any preceding clause wherein the at least one imaging modality is: computed tomography, fluoroscopy, magnetic resonance imaging, mammography, x-ray, positron emission tomography, or ultrasound.
31. The kit of any preceding clause wherein the imaging structure comprises a series of gradations.
32. The kit of any preceding clause wherein the series of gradations comprises a distance scale.
33. The kit of any preceding clause wherein the insertion tip comprises a sharpened tip.
34. The kit of any preceding clause wherein the insertion tip comprises a beveled tip to form the sharpened tip.
35. The kit of any preceding clause wherein the needle lumen is marginally larger than the diameter of the guide wire to provide a sliding fit clearance between the guide wire and the introduction needle.
36. The kit of any preceding clause further comprising a drainage catheter assembly defining a catheter lumen greater than the diameter of the guide wire.
37. The kit of any preceding clause wherein the catheter comprises a flexible, hollow body terminating in a distal end comprising a tapered tip, and the hollow body defining the catheter lumen with a tip outlet in the tapered tip.
38. The kit of any preceding clause wherein the catheter lumen has a portion at the tip outlet marginally larger than the diameter of the guide wire to provide a sliding fit clearance for the guide wire.
39. The kit of any preceding clause further comprising a hollow stiffening rod slidably received within at least a portion of the catheter lumen and defining a stiffening rod lumen greater than the diameter of the guide wire.
40. The kit of any preceding clause wherein the catheter comprises at least one fluid port located in a hollow body at the distal end.
41. The kit of any preceding clause wherein the distal end of the catheter comprises a curled portion.
42. The kit of any preceding clause wherein the curled portion is straightened when the stiffening rod is received within the catheter lumen.
43. The kit of any preceding clause wherein the at least one fluid port is located in the curled portion.
44. The kit of any preceding clause wherein at least the tapered tip of the catheter comprises a lubricious coating.
45. A method of draining a site in a tissue mass, the method comprising imaging the tissue mass to locate the site, while imaging the site in the tissue mass, non-intravenously introducing a guide wire to the site, where the guide wire diameter is 0.018 inch (0.46 millimeters) or smaller, dilating the tissue mass by sliding a dilator of 4 to 10.5 French, with a dilator lumen of marginally larger than the diameter of the guide wire, over the guide wire; and placing a drainage catheter assembly of 4 to 10.5 French at the site, with a catheter lumen marginally larger than the diameter of the guide wire, by sliding the drainage catheter assembly over the guide wire and to the site.
46. The method of any preceding clause wherein the dilator is removed from the tissue mass prior to the sliding of the drainage catheter assembly over the guide wire.
47. The method of any preceding clause wherein the dilator is a sheath dilator comprising a dilator and a surrounding sheath and the dilating the tissue mass comprises removing the dilator while leaving the sheath and the placing the drainage catheter assembly comprises sliding the drainage catheter assembly into the sheath.
48. The method of any preceding clause comprising removing the sheath after the placing of the drainage catheter assembly.
49. The method of any preceding clause wherein the sliding the drainage catheter assembly is done during imaging of the tissue mass.
50. The method of any preceding clause wherein the sliding the drainage catheter assembly is done while imaging the tissue mass.
51. The method of any preceding clause wherein the sliding the drainage catheter assembly while imaging comprises sliding the drainage catheter assembly until an imaging structure on a catheter is at a desired location relative to the site based on the imaging.
52. The method of any preceding clause further comprising removing a hollow stiffening rod from the drainage catheter assembly after the placing of the drainage catheter assembly.

When used in this specification and claims, the terms "comprises" and "comprising" and variations thereof mean that the specified features, steps or integers are included. The terms are not to be interpreted to exclude the presence of other features, steps or components.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

Although certain example embodiments of the invention have been described, the scope of the appended claims is not intended to be limited solely to these embodiments. The claims are to be construed literally, purposively, and/or to encompass equivalents.

## Claims

1. A drainage catheter assembly (10) comprising:
a catheter (12, 64, 212, 312), comprising a flexible, hollow body (20, 84, 220, 362) terminating in a distal end (18, 88, 218, 318) comprising a tapered tip (22, 90, 222, 322) and the hollow body (20, 84, 220, 362) defining a catheter lumen (21, 100, 221, 321) with a tip outlet (28, 96, 228, 328) in the tapered tip (22, 90, 222, 322);
a hollow stiffening rod (14, 82) slidably received within the catheter lumen (21, 100, 221, 321) and defining a stiffening rod lumen (44, 108) marginally greater than 0.455 millimeters (0.018 inches);
the catheter lumen (21, 100, 221, 321) having a first portion (34, 102, 234, 321) sized to slidably receive the stiffening rod (44, 108) and a second portion (36, 106, 236, 336), at least including the tip outlet (28, 96, 228, 328), and marginally greater than 0.455 millimeters (0.018 inches); and
at least one fluid port (40, 240, 340) located in the hollow body (20, 84, 220, 362) at the distal end (18, 88, 218, 318).

2. The drainage catheter assembly (10) of claim 1 wherein the at least the distal end (18, 88, 218, 318) comprises a curled portion (120, 320).

3. The drainage catheter assembly (10) of claim 2 wherein the curled portion (120, 320) is straightened when the stiffening rod (14, 82) is received within the catheter lumen (21, 100, 221, 321).

4. The drainage catheter assembly (10) of claim 3 wherein the at least one fluid port (40, 240, 340) is located in the curled portion (120, 320).

5. The drainage catheter assembly (10) of any of claims 1-4 where in the first portion (34, 102, 234, 321) extends into the tapered tip (22, 90, 222, 322).

6. The drainage catheter assembly (10) of any of claims 1-5 wherein the at least one fluid port (40, 240, 340) directly fluidly couples to the first portion (34, 102, 234, 321) of the catheter lumen (21, 100, 221, 321).

7. The drainage catheter assembly (10) of any of claims 1-6 wherein the first portion (34, 102, 234, 321) has a larger inner diameter than the second portion (36, 106, 236, 336).

8. The drainage catheter assembly (10) of any of claims 1-7 wherein at least the tapered tip (22, 90, 222, 322) comprises a lubricious coating.

9. The drainage catheter assembly (10) of any of claims 1-8 wherein the tapered tip (22, 90, 222, 322) tapers continuously.

10. The drainage catheter assembly (10) of any of claims 1-9 further comprising a 0.018 inch (0.46 millimeters) diameter guide wire (60) that can be slidably received through the first and second portion (34, 36, 102, 106, 234, 236, 321, 336).

11. The drainage catheter assembly (10) of claim 10 wherein the guide wire (60) comprises a guide wire distal end (66) that is curled (68), soft, shapeable, or flexible, and made from a shape-memory material.

12. The drainage catheter assembly (10) of any of claims 1-11 further comprising an imaging structure (50) provided on at least one of the catheter (12, 64, 212, 312) or stiffening rod (14, 82), with the imaging structure (50) having at least one imaging modality.

13. The drainage catheter assembly (10) of claim 12 wherein the at least one imaging modality is: computed tomography, fluoroscopy, magnetic resonance imaging, mammography, x-ray, positron emission tomography, or ultrasound.

14. The drainage catheter assembly (10) of any of claims 12-13 wherein the imaging structure (50) comprises a series of gradations.

15. The drainage catheter assembly (10) of claim 14 wherein the series of gradations comprises a distance scale.
